Europäisches Patentamt

European Patent Office

Office européen des brevets

(19)

(11) Veröffentlichungsnummer: **0 009 789**
**B1**

(12) **EUROPÄISCHE PATENTSCHRIFT**

(45) Veröffentlichungstag der Patentschrift: 01.09.82

(21) Anmeldenummer: **79103719.5**

(22) Anmeldetag: **01.10.79**

(51) Int. Cl.³: **A 61 B 5/02, A 61 B 17/12** ·

(54) Manschette für Sphygmomanometer.

(30) Priorität: **06.10.78 DE 2843643**

(43) Veröffentlichungstag der Anmeldung:
**16.04.80 Patentblatt 80/8** ·

(45) Bekanntmachung des Hinweises auf die Patenterteilung:
**01.09.82 Patentblatt 82/35** ·

(84) Benannte Vertragsstaaten:
**AT BE CH DE FR GB IT LU NL SE** ·

(56) Entgegenhaltungen:
**DE - A - 67 543**
**DE - A - 1 541 147**
**DE - C - 566 052**
**FR - A - 777 690**
**US - A - 3 633 567**
**US - A - 3 713 446**
**US - A - 4 106 499**

(73) Patentinhaber: **BOEHRINGER MANNHEIM GMBH**
**Sandhofer Strasse 112-132 Postfach 31 01 20**
**D-6800 Mannheim 31-Waldhof (DE)** ·

(72) Erfinder: **Rebbe, Klaus**
**Bodelschwinghweg 23**
**D-6800 Mannheim 31 (DE)**
Erfinder: **Wellmann, Klaus**
**Meerfeldstrasse 46**
**D-6800 Mannheim 1 (DE)**

Courier Press, Leamington Spa, England.

## Manschette für Sphygmomanometer

Die Erfindung betrifft eine Manschette für Sphygmomanometer, umfassend ein schlauchartiges Manschetenband, eine Schnalle mit einem ersten Bügel und einem zweiten Bügel, wobei an dem ersten Bügel das eine Ende des Bandes befestigt ist und das andere freie Ende des Bandes eine Schlinge bildend zwischen den Bügeln hindruchgeführt ist und ein Paar von Belägen, die bei Berührung miteinander eine lösbare Haftverbindung bilden, zur vorübergehenden Festlegung der Schlinge, wobei der erste Belag auf dem Manschettenband angeordnet ist.

Derartige Manschetten sind beispielsweise bekannt aus der US—A—4 106 499. Die Schnalle dieser bekannten Einrichtung ist aus einem zu einem langgestreckten oval gebogenen Metallstab hergestellt. An dem einen Bügel der Schnalle, der in diesem Fall durch den einen Schenkel des Metallstabs gebildet wird, ist das Manschettenband dauerhaft befestigt. Auf dem Manschettenband befinden sich die beiden Beläge, die bei Berührung miteinander eine lösbare Haftverbindung bilden. Im häufigsten Fall werden diese beiden Beläge, wie auch bei der US—A—4 106 499, als Klettenbeläge ausgebildet, wobei der eine Belag im allgemeinen als "Flausch", der andere Belag als "Häkchen" bezeichnet wird. Beide Beläge müssen, wie in der zitierten Druckschrift dargestellt wird, in Längsrichtung des Bandes einen Mindestabstand voneinander haben, um das Anlegen der Manschette an Patienten mit verschieden dicken Gliedmaßen zu ermöglichen.

Die bekannten Manschetten können mit einer Hand nur sehr schwierig angelegt werden. Dies ist insbesondere darauf zurückzuführen, daß das Manschettenband bei den bekannten Einrichtungen zunächst mit der freien Hand angezogen werden muß und danach ein Umgreifen notwendig ist, um die beiden Beläge aufeinander festzulegen.

Um die Problematik der Einhand-Bedienung zu lösen, wird in der US—A—3 633 567 eine Einrichtung vorgeschlagen, die sich insbesondere durch Verwendung einer Blattfeder auszeichnet, die für eine Vorspannung des Manschettenbandes sorgt und dadurch das Anlegen der Manschette mit einer Hand erleichtern soll. Diese vorbekannte Einrichtung ist jedoch sehr kompliziert im Aufbau und demzufolge kostspielig in der Herstellung. Außerdem hat sie einen erheblichen Raumbedarf und läßt sich deswegen verhältnismäßig schwer verpacken und transportieren, was gerade bei Geräten zur Selbstmessung des Blutdrucks ein wesentlicher Faktor ist.

Aufgabe der vorliegenden Erfindung ist es daher, eine in der Konstruktion einfache und kostengünstig herzustellende mit einer Hand leicht bedienbare Manschette zu schaffen, die der Benutzer ohne fremde Hilfe selbst am eigenen Arm anlegen kann. Eine solche Manschette soll genauso leicht mit einer Hand wieder gelöst werden können.

Diese Aufgabe wird bei einer Manschette der eingangs genannten Art dadurch gelöst, daß der zweite Belag auf dem zweiten Bügel angeordnet ist.

Die vorteilhafte Wirkungsweise der erfindungsgemäßen Konstruktion ist insbesondere darauf zurückzuführen, daß der auf dem Bügel der Schnalle angeordnete Belag, also beispielsweise die Häkchen eines Klettenverschlusses, unabhängig von der Armdicke des Patienten sich jeweils in einer bestimmten Position bezogen auf das an der Schnalle befestigte Ende der Manschette befindet. Der andere Teil des Belagpaares, beispielsweise also der Flausch eines Klettenverschlusses, kann sich über die gesamte Länge des Manschettenbandes erstrecken. In Folge dieser Verhältnisse wird die Schnalle bereits befestigt, wenn der Benutzer das Band anzieht und über die Schnalle hinweglegt. Ein zwischenzeitiges Loslassen des freien Endes und Umgreifen ist somit nicht mehr notwendig.

Vorteilhaft erstrecken sich die Beläge etwa über die gesamte Breite des Bandes und des Bügels. Außer den erwähnten Klettenbelägen kommen insbesondere auch magnetische beziehungsweise magnetisierbare Beläge in Betracht, wie sie—befestigt auf dem Manschettenband—aus der DD—A—67 543 bekannt sind.

Die Einhand-Bedienung der erfindungsgemäßen Manschette wird besonders erleichtert durch eine bevorzugte Ausführungsform, gemäß der der den zweiten Belag tragende zweite Bügel der Schnalle wesentlich größer als der erste Bügel ist und eine in seiner Gesamtheit—sei es kontinuierlich oder als gegeneinander gewinkelte Enzelflächenkonvex gekrümmte Oberfläche hat, dergestalt, daß der zweite Belag einen von der Stelle, an der das Band zwischen den Bügeln hindurchgeführt, ist, aus zunehmenden Abstand von dem Glied des Benutzers hat, an den die Manschette angelegt wird. Eine besonders bevorzugte Ausführungsform insbesondere dieser Version ist dadurch gekennzeichnet, daß der zweite Bügel im Querschnitt halbrund geformt ist, wobei der Durchmesser des entsprechenden Kreises um ein Vielfaches größer als der Durchmesser des ersten Bügels ist. Eine derartige Ausführungsform erlaubt darüber hinaus eine möglichst reibungsfreie Durchführung des Bandes.

Es hat sich ferner als vorteilhaft erwiesen, daß die Schnalle mindestens halb so schwer wie das Band ist, damit sie beim Öffnen der Schlinge das Band so nach sich zieht, daß sich die Manschette allein durch das Gewicht der Schnalle öffnet.

Weitere vorteilhafte Ausgestaltungen der Erfindung werden an Hand der in den Zeichnungen dargestellten Ausführungsbeispiele näher erläutert. Es zeigen:

Fig. 1 in dreidimensionaler Ansicht eine um einen nicht dargestellten Arm geschlossene Manschette

Fig. 2 und 3 Querschnitte andere Versionen der Schnalle

Fig. 4 verschiedene Schritte beim Anlegen und Abnehmen der Manschette.

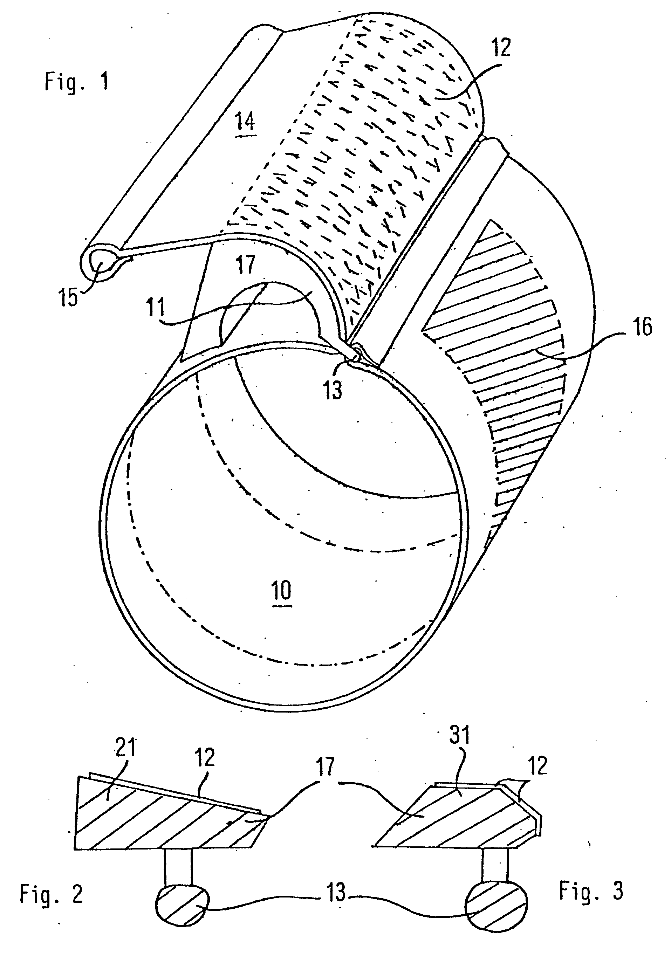

Das freie Ende der in Fig. 1 dargestellten Manschette 10 ist durch den Bügel geschoben und gegen Herausrutschen mit einer Rolle 15 gesichert. Es liegt also eine Schlinge vor, die zur Blutdruckmessung Lufträume enthält, mit denen im geschlossenen Zustand der Druck auf den umfassten Körperteil erhöht werden kann.

Das besondere der Erfindung liegt an dem relativ großen Bügel 17 der Schnalle gegenüber dem kleinen Schnallenbügel 13. Auf dem Bügel 17 der Schnalle 11 ist ein Belag 12 angebracht, der an dem Belag 16 der Manschette 10 haftet, wenn beide aufeinander gelegt werden.

Üblicherweise werden, wie bereits eingangs erwähnt, Stoffbeläge verwendet, die aufeinander klettenartig haften und die bei den bekannten Einrichtungen beide auf dem Manschettenband angebracht sind. Diese müssen, wie in der eingangs zitierten US—A— 4 106 499 beschrieben, einen bestimmten Mindestabstand voneinander haben. Dadurch ergibt sich eine Übergangsstelle der Manschette 10, die die Manschette als solches schwächt und zu Verschlingungen oder Verklemmungen führen kann, was bei der Erfindung dadurch vermieden wird, daß eine Art des klettenartigen Stoffes auf den Bügel 17 verlegt worden ist.

Der Hauptvorteil der erfindungsgemäßen Einrichtung besteht aber in der erleichterten Einhand-Handhabung, bei der kein Umgreifen mehr erforderlich ist.

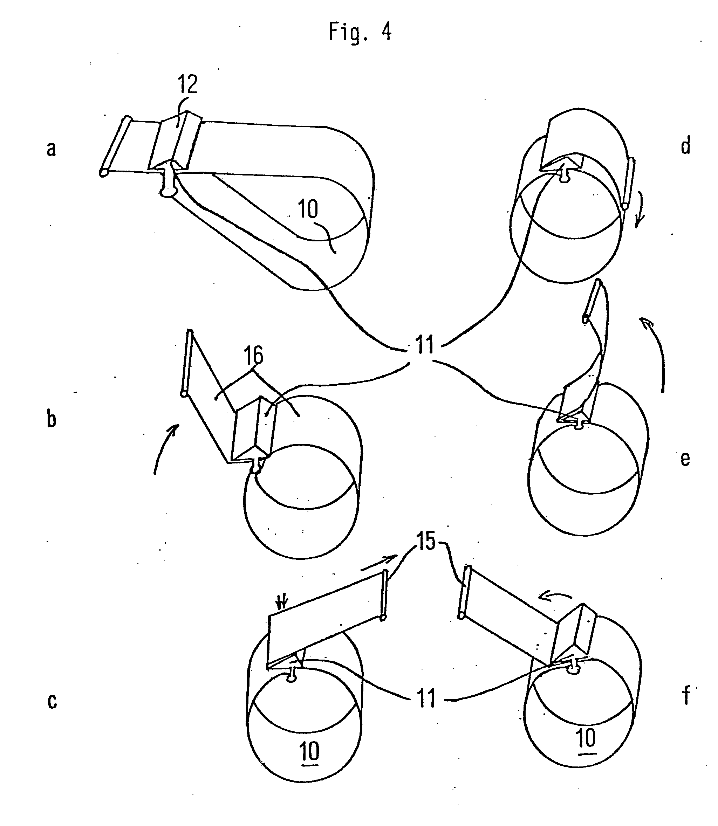

Die Handhabe der in Fig. 1 dargestellten Manschette ist in Fig. 4 in Zwischenschritten dargestellt. Grundsätzlich können verschiedene Bügelformen 21, 31, wie in Fig. 2 und 3 dargestellt, verwendet werden.

Zum Anlegen wird in Grundstellung Fig. 4a die Manschette über den Arm gestülpt und mit der Rolle in einer Richtung angezogen. Zum Anziehen der Schlinge wird die Zugrichtung verändert (Fig. 4b). Die Schnalle 11 rutscht schlüssig an das einzuschnürende Glied.

In dieser Lage ist die Manschette zu befestigen, wozu das freie Ende mit der Rolle 15 über den Bügel 17 der Schnalle 11 gelegt wird. Die Schnalle ist bereits befestigt, ohne nach zwischenzeitigem Loslassen das freie Ende von unten nachzuziehen. Das freie Ende fällt lose herab (Fig. 4d), die Manschette ist betriebsbereit.

Zum Lösen der Manschette wird das freie Ende 15 der Manschette auf dem gleichen Weg zurückgezogen (Fig. 4e).

Beim Zurückziehen ist zunächst der Haft-Widerstand zwischen den Belägen zu überwinden (Fig. 4f). Danach ist die Schlinge der Manschette wieder gelockert.

Da die Schnalle 11 vorteilhaft mindestens halb so schwer wie die gesamte Manschette 10 ist, zieht die Schnalle 11 durch ihr Eigengewicht die Schlinge wieder in eine geöffnete Stellung entsprechend Fig. 4a. Die Manschette kann wieder leicht vom Arm abgestreift werden.

Diese einfache Bedienungsweise macht deutlich, daß die neue Manschette problemlos mit einer Hand zu befestigen ist und sich somit vorzüglich für Sphygmomanometer eignet, die ohne fremde Hilfe (also ohne Beisein eines Arztes) angelegt werden können.

## Patentansprüche

1. Manschette für Sphygmomanometer, umfassend ein schlauchartiges Manschettenband (10), eine Schnalle (11) mit einem ersten Bügel (13) und einem zweiten Bügel (17), wobei an dem ersten Bügel (13) das eine Ende des Bandes (10) befestigt ist und das andere freie Ende des Bandes (10), eine Schlinge bildend, zwischen den Bügeln (13, 17) hindurchgeführt ist, und ein Paar von Belägen (12, 16), die bei Berührung miteinander eine lösbare Haftverbindung bilden, zur vorübergehenden Festlegung der Schlinge, wobei der erste Belag (16) auf dem Manschettenband angeordnet ist, dadurch gekennzeichnet, daß der zweite Belag (12) auf dem zweiten Bügel (17) angeordnet ist.

2. Manschette nach Anspruch 1, dadurch gekennzeichnet, daß die Beläge (12, 16) sich etwa über die gesamte Breite des Bandes und des Bügels erstrecken.

3. Manschette nach Anspruch 1 oder 2, dadurch gekennzeichnet, daß die Beläge (12, 16) klettenartig zusammenhaftende Beläge sind.

4. Manschette nach Anspruch 1 oder 2, dadurch gekennzeichnet, daß die Beläge (12, 16) magnetische bzw. magnetisierbare Beläge sind.

5. Manschette nach einem der Ansprüche 1 bis 4, dadurch gekennzeichnet, daß der den zweiten Belag (12) tragende zweite Bügel (17) der Schnalle (11) wesentlich größer als der erste Bügel (13) ist und eine in ihrer Gesamtheit konvex gekrümmte Oberfläche hat, dergestalt, daß der zweite Belag einen von der Stelle, an der das Band (10) zwischen den Bügeln (13, 17) hindurchgeführt ist, aus zunehmenden Abstand von dem Glied des Benutzers hat, an den die Manschette angelegt wird.

6. Manschette nach einem der vorhergehenden Ansprüche dadurch gekennzeichnet, daß der dem zweiten Belag (12) tragende zweite Bügel (17) im Querschnitt halbrund geformt ist, dessen Durchmesser um ein Vielfaches größer als der Durchmesser des ersten Bügels (13) ist.

7. Manschette nach einem der vorhergehenden Ansprüche, dadurch gekennzeichnet, daß der den zweiten Belag (12) tragende zweite Bügel (17) eine etwa über die gesamte Breite des Bandes (10) plane Fläche hat.

8. Manschette nach einem der vorhergehenden Ansprüche, dadurch gekennzeichnet, daß die Schnalle (11) mindestens halb so schwer wie das Band (10) ist.

## Revendications

1. Manchette pour sphygmomanomètre, comprenant une bande (10) en forme de tube, une boucle (11) comportant une première bride (13) et une deuxième bride (17), une extrémité de la bande (10) étant fixée à la première bride (13), l'autre extrémité libre de la bande (10), formant un lacet, passant entre les brides (13, 17), et un jeu de garnitures (12,16) formant ensemble une liaison adhésive lorsqu'elles entrent en contact afin de fixer provisoirement le lacet, la première garniture (16) étant disposée sur la bande formant la manchette, caractérisée en ce que la deuxième garniture (12) est disposée sur la deuxième bride (17).

2. Manchette selon la revendication 1 caractérisée en ce que les garnitures (12, 16) s'étendent environ sur toute la largeur de la bande et de la bride.

3. Manchette selon la revendication 1 ou 2, caractériséee en ce que les garnitures (12, 16) adhèrent ensemble par accrochage.

4. Manchette selon la revendication 1 ou 2, caractérisée en ce que les garnitures (12, 16) sont magnétiques ou magnétisables.

5. Manchette selon l'une des revendications 1 à 4, caractérisée en ce que la second bride (17) de la boucle (11), comportant la seconde garniture (12), est essentiellement plus grande que la première bride (13) et présente, vue dans son ensemble, une surface convexe, de telle façon que la deuxième garniture soit éloignée d'une distance croissante du membre de l'utilisateur auquel la manchette est appliquée, comptée à partir de l'endroit où la bande (10) passe entre les brides (13, 17).

6. Manchette selon l'une quelconque des revendications précédentes, caractérisée en ce que la seconde bride (17) comportant la seconde garniture (12) présente en coupe transversale la forme d'une demicercle dont le diamètre est plusieurs fois plus grand que le diamètre de la première bride (13).

7. Manchette selon l'une quelconque des revendications précédentes, caractérisée en ce que la deuxième bride (17) comportant la deuxième garniture (12) présente une face plane s'étendant sur presque toute la largeur de la bande (10).

8. Manchette selon l'une quelconque des revendications précédentes, caractérisée en ce quel le poids de la boucle (11) est au moins égal à la moitié du poids de la bande (10).

## Claims

1. Cuff for a sphygomanometer, comprising a tube-like cuff band (10), a buckle (11) with a first clamp member (13) and a second clamp member (17), whereby on the first clamp member (13) there is fixed one end of the band (10) and the other free end of the band (10) forming a loop, is passed through between the clamp members (13, 17) and a pair of coverings (12, 16) which, in the case of contacting with one another, form a releasable holding connection for the temporary securing of the loop, whereby the first covering (16) is arranged on the cuff band, characterised in that the second covering (12) is arranged on the second clamp member (17).

2. Cuff according to claim 1, characterised in that the coverings (12, 16) extend approximately over the total breadth of the band and of the clamp member.

3. Cuff according to claim 1 or 2, characerised in that the coverings (12, 16) are coverings holding together in a burr-like manner.

4. Cuff according to claim 1 or 2, characterised in that the coverings (12, 16) are magnetic or magnetisable coverings.

5. Cuff according to one of claims 1 to 4, characterised in that the second clamp member (17) of the buckle (11) carrying the second covering (12) is substantially larger than the first clamp member (13) and, in its totality, has a convexly-curved surface such that the second covering has a distance from the member of the user about which the cuff is placed which increases from the place at which the band (10) is passed through between the clamp members (13, 17).

6. Cuff according to one of the preceding claims, characterised in that the second clamp member (17) carrying the second covering (12) is formed semicircularly in cross-section, the diameter of which is a multiple larger than the diameter of the first clamp member (13).

7. Cuff according to one of the preceding claims, characterised in that the second clamp member (17) carrying the second covering (12) has a plane surface over approximately the whole breadth of the band (10).

8. Cuff according to one of the preceding claims, characterised in that the buckle (11) is at least half as heavy as the band (10).

Fig. 1

14

12

15

17

11

13

16

10

21

12

17

31

12

Fig. 2

13

Fig. 3

## Fig. 4